# EUROPÄISCHE PATENTSCHRIFT

(11) **EP 0 668 344 B1**
(45) Veröffentlichungstag und Bekanntmachung des Hinweises auf die Patenterteilung: **17.05.2000**
(21) Anmeldenummer: 94120636.9
(22) Anmeldetag: 24.12.1994
(51) Int. Cl.: C11D 3/12, C11D 3/20, C07C 59/305

(54) **Polyepoxy-Bernsteinsäure enthaltende Waschmittel**
Detergent composition containing polyepoxy succinic acid
Composition de détergents contenant de l'acide polyépoxy-succinique

(30) Priorität: 11.02.1994 DE 4404333
(43) Veröffentlichungstag der Anmeldung: 23.08.1995
(73) Patentinhaber: BK Giulini Chemie GmbH, 67029 Ludwigshafen (DE)
(72) Erfinder: Himmrich, Johannes, Dr., D-50354 Hürth (DE); Gohla, Werner, D-53859 Niederkassel (DE); Krämer, Albrecht, Dr., D-68775 Ketsch (DE); Klein, Thomas, Dr., D-69121 Heidelberg (DE); Merkenich, Karl, Dr., D-64658 Fürth-Fahrenbach (DE)
(74) Vertreter: Grussdorf, Jürgen, Dr.

(56) Entgegenhaltungen:
- EP-A- 0 267 371
- WO-A-92/07928
- WO-A-92/18594
- US-A- 4 654 159

## Beschreibung

Zur Erzielung einer guten Wasch- und Reinigungsleistung werden modernen synthetischen, phosphatfreien Waschmitteln bestimmte Waschhilfsstoffe zugesetzt, die das Schmutztragevermögen der Waschflotte erhöhen und damit die Inkrustierungsneigung erniedrigen. Als solche Waschhilfsstoffe werden dazu zur Zeit monomere, oligomere oder polymere Polycarboxylate verwendet. Nachteil dieser Stoffe ist, daß sie kaum biologisch abbaubar sind.

Als Neuentwicklung auf dem Gebiet der Gerüststoffe, auch Builder genannt, werden in der EP-B1-0 164 514 kristalline schichtförmige Natriumsilikate der Zusammensetzung NaMSiₓO₂ₓ₊₁ . zH₂O, wobei M Natrium oder Wasserstoff bedeuten und x eine Zahl von 1,9 bis 4 und z eine Zahl von 0 bis 20 ist, vorgestellt. Aus der WO 92-18594 ist ferner bekannt, zusätzlich zu 10 bis 95 % Schichtsilikaten 5 bis 90 % einer Polycarbonsäure wie Ascorbinsäure, Citronensäure oder Bernsteinsäure als Buildersubstanz zu verwenden.

Aus der WO 92-07928 ist bekannt, eine Mischung aus Zeolith, polymerem Polycarboxylat und Schichtsilikat in Wasch- und Reinigungsmitteln zu verwenden, wobei als Polycarboxylat Polymere von Acrylsäure, Methacrylsäure oder Maleinsäure eingesetzt werden. Nachteilig an diesen Mischungen ist die schlechte biologische Abbaubarkeit der organischen Säuren.

Aufgabe der vorliegenden Erfindung war es, einen Waschhilfsstoff zu finden, der die Inkrustierungsneigung eines Waschmittels auf Basis Natriumschichtsilikat bzw. Natriumschichtsilikat/Zeolith noch weiter erniedrigt und gleichzeitig besser biologisch abbaubar ist als die bekannten Polycarboxylate.

Diese Aufgabe konnte durch Verwendung von Polyepoxybernsteinsäure (PEBS) als Waschhilfsstoff in Waschmitteln auf Basis Natriumschichtsilikat bzw. Natriumschichtsilikat/Zeolith als Gerüststoffe gelöst werden.

Aus der DE-0S 24 08 591 ist die Herstellung einer Polyepoxybernsteinsäure sowie ihre Verwendung als Gerüststoff in Wasch- und Reinigungsmitteln bekannt.

In der US-PS 4,654,159 wird der Einsatz einer PEBS (= PESA) als Metallkomplexbildner anstelle von Polyphosphaten in Waschmitteln auf Basis
- - Natriumcarbonat: (Mengenverhältnis Gerüststoff : PEBS = 17:25)
- - Natriumcarbonat/Zeolith: A (Mengenverhältnis Gerüststoff : PEBS = 30: 7)
- - Natriumtripolyphosphat: (Mengenverhältnis Gerüststoff : PEBS = 12:10)
beschrieben (vgl. besonders Spalten 18 und 19). Eine Kombination mit Schichtsilikaten wird nicht genannt.

Aus der EP 0 267 371 A1 sind Geschirrspülmittel bekannt, welche Schichtsilikate und Polycarboxylate, insbesondere Maleinsäurecopolymerisate, als Buildersubstanzen enthalten. Die Kombination haftet nicht auf den Geschirroberflächen.

Überraschenderweise wurde gefunden, daß bei Waschmitteln auf Basis Natriumschichtsilikat bzw. Natriumschichtsilikat/Zeolith als Gerüststoffe schon mit deutlich reduziertem PEBS-Gehalt, verglichen mit dem Stand der Technik gemäß der US-PS 4,654,159, extrem niedrige Inkrustierungswerte (Gewebeaschen) erhalten werden, d.h. sich die Schichtsilikate nicht in die Gewebe einlagern.

Im einzelnen betrifft die Erfindung ein pulverförmiges Waschmittel, enthaltend 0,5 bis 60 Gew% Gerüststoffe aus der Substanzklasse der Natriumschichtsilikate der Formel NaMSiₓO₂ₓ₊₁ . zH₂O mit M = Na oder H; x = 1,9 bis 4; und z = 0 bis 20, gegebenenfalls in Kombination mit Zeolith A; 5 bis 50 Gew% mindestens eines Tensids; 0,5 bis 10 Gew% mindestens einer Polyepoxybernsteinsäure der Formel 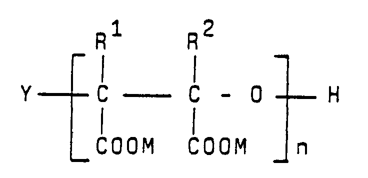 mit R¹, R² = H, C₁₋₄-Alkyl, substituiertes C₁₋₄-Alkyl; M = H, Na, K, NH₄ oder substituiertes Ammonium; Y = -OH, -OR', -NHR', -NR'₂, -SR', -SO₃M (M = H, Na, K), -PR'₂ mit R' = Alkyl, substituiertes Alkyl, Aryl oder substituiertes Aryl; n = 2 bis 15; und als Rest übliche Waschhilfsstoffe.

Darüberhinaus kann das Waschmittel der Erfindung bevorzugt und wahlweise dadurch gekennzeichnet sein, daß
a) der Gehalt an Gerüststoffen 5 bis 60 Gew%, an Tensiden 10 bis 30 Gew% und an Polyepoxybernsteinsäure 0,5 bis 5 Gew% beträgt;
b) das Gewichtsverhältnis Gerüststoffe zu Polyepoxybernsteinsäure 5 : 1 bis 100 : 1 beträgt;
c) in der Formel der Polyepoxybernsteinsäure n = 2 bis 10, insbesondere 2 bis 4 bedeutet;
d) als Gerüststoff aus der Substanzklasse der Natriumschichtsilikate δ-Na₂Si₂O₅ eingesetzt wird.

Im Vergleich zu den zur Zeit verwendeten Waschhilfsstoffen auf Basis von Polycarboxylaten zeichnet sich eine Polyepoxybernsteinsäure obiger Formel durch eine verbesserte biologische Abbaubarkeit aus. Polyepoxybernsteinsäure gilt als toxikologisch unbedenklich und verfügt über ein hohes Bindevermögen für Erdalkaliionen. Außerdem besitzt Polyepoxybernsteinsäure eine korrosionsinhibierende Wirkung im Gegensatz zu vergleichbaren Substanzen auf Basis von Polycarboxylaten.

Die biologische Abbaubarkeit wurde nach OECD 302 (Zahn-Wellens-Test) und OECD 303 A (Coupled-Units-Test) an einer Polyepoxybernsteinsäure mit Y = OH und R¹, R² = H; M = K und n = 2 bis 15, Maximum der Verteilung bei n = 2 bis 4, in der Folge als Typ A bezeichnet, bestimmt.

**TABELLE 1**

| Testverfahren | biologische Abbaubarkeit |
|---|---|
| OECD 302 | 60 % |
| OECD 303 A | 65 % |

Außerdem wurde die Belebtschlammadsorption und Fällbarkeit mit Eisen-II-sulfat der oben angeführten Polyepoxybernsteinsäure Typ A untersucht. Dabei zeigte sich, daß diese Polyepoxybernsteinsäure zu 100 % adsorbierbar und fällbar ist.

Die Fisch- und Bakterientoxizität obiger Polyepoxybernsteinsäure Typ A zeigt die nachfolgende Tabelle.

**TABELLE 2**

| Prüfparameter | |
|---|---|
| Fischtoxizität | : LC 50 (Zebrabärbling) > 1.000 mg/l |
| Bakterientoxizität | : EC 50 > 10.000 mg/l |

Das Calciumbindevermögen der Polyepoxybernsteinsäure und ihrer Derivate wurde bei 20 °C und einem pH-Wert von 10 durch Titration von frisch gefälltem CaCO₃ bestimmt.

Die nachfolgend aufgeführten Typen B, C und D gleichen Typ A mit folgenden Unterschieden:
- Typ B:: Y = -NH-CH₂-CH₂-OH
- Typ C:: Y = -NH-CH₂-COOK (Glycyl)
- Typ D::

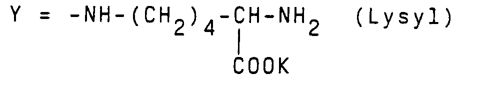

**TABELLE 3**

| Substanz | mg CaO/g |
|---|---|
| Typ A | 224 |
| Typ B | 234 |
| Typ C | 175 |
| Typ D | 155 |

Das Korrosionsverhalten in Gegenwart von Polyepoxybernsteinsäure Typ A wurde nach der Kuponmethode ermittelt (wlb "Wasser, Luft und Betrieb" 1-2/80, S. 42-44, (1980), Korrosionsversuche in Kaltwasser mit Stahlkupons und anorganischen Phosphaten als Korrosionsinhibitoren). Die erhaltenen Ergebnisse sind in der nachfolgenden Tabelle 4 zusammengestellt.

**TABELLE 4**

| Korrosionsverhalten von Stahlblech ST37, Werkstoff-Nr. 1.0110 in natürlichem Leitungswasser in Gegenwart von Polyepoxybernsteinsäure (PEBS) Typ A | | | | | | |
|---|---|---|---|---|---|---|
| | Korrosionsrate in Millimeter pro Jahr (mm/a) . 10⁻³ | | | | | |
| Versuchszeit in Stunden | 2 | 24 | 48 | 120 | 144 | 168 |
| 0 ppm PEBS | 279 | 163 | 125 | 102 | 102 | 89 |
| 200 ppm PEBS | 51 | 13 | 13 | 13 | 13 | 13 |
| 300 ppm PEBS | 51 | 15 | 15 | 13 | 13 | 13 |
| 400 ppm PEBS | 51 | 18 | 18 | 10 | 5 | 5 |

Bekanntlich können aus dem Erdalkalibindevermögen einer Substanz allein keine sicheren Rückschlüsse auf die waschtechnischen Eigenschaften dieser Substanz gezogen werden. Auch die Literaturhinweise über die Wascheigenschaften von Polyepoxybernsteinsäure in Kombination mit Natriumcarbonat, Zeolith A/Natriumcarbonat oder Natriumtripolyphosphat (US-PS 4,654,159) geben keinen Anhaltspunkt über die Verwendbarkeit von PEBS in Kombination mit Schichtsilikaten der Formel NaMSiₓO₂ₓ₊₁ . zH₂O in Wasch- und Reinigungsmitteln.

In Waschversuchen wurde aber überraschenderweise gefunden, daß trotz andersartigem Eigenschafts- und Anwendungsprofil von Schichtsilikaten der Formel NaMSiₓO₂ₓ₊₁ . zH₂O mit PEBS als Waschhilfsstoff die Inkrustierungsneigung eines Waschmittels auf Basis dieser Natriumschichtsilikate, gegebenenfalls in Kombination mit Zeolith A, deutlich verringert werden kann. Dabei werden schon mit unerwartet niedrigen PEBS-Gehalten, verglichen mit dem Stand der Technik gemäß der US-PS 4,654,159, extrem geringe Inkrustierungswerte (Gewebeaschen) erhalten.

Zu den Tensiden und den Waschhilfsstoffen im pulverförmigen Waschmittel der Erfindung sei noch folgendes ausgeführt:

Unter anionischen Tensiden sind die wasserlöslichen Salze höherer Fettsäuren oder Harzsäuren, wie Natrium- oder Kaliumseifen von Kokos-, Palmkern- oder Rüböl sowie von Talg und Gemischen davon zu verstehen. Weiterhin zählen dazu höhere alkylsubstituierte, aromatische Sulfonate, wie lineare Alkylbenzolsulfonate mit 9 bis 14 C-Atomen im Alkylrest (LAS), Alkylnaphthalinsulfonate, Alkyltoluolsulfonate, Alkylxylolsulfonate oder Alkylphenolsulfonate; Fettalkoholsulfate (R-CH₂-O-SO₃Na; R = C₁₁₋₁₇) oder Fettalkoholethersulfate, wie Alkalilaurylsulfat oder Alkalihexadecylsulfat, Triethanolaminlaurylsulfat, Natrium- oder Kaliumoleylsulfat, Natrium- oder Kaliumsalze von mit 2 bis 6 Mol Ethylenoxid ethoxyliertem Laurylsulfat. Weitere geeignete anionische Tenside sind sekundäre lineare Alkansulfonate sowie α-Olefinsulfonate mit einer Kettenlänge von 12 bis 20 C-Atomen.

Unter nichtionischen Tensiden (Nonionics) sind solche Verbindungen zu verstehen, die eine organische, hydrophobe Gruppe sowie einen hydrophilen Rest aufweisen, z.B. die Kondensationsprodukte von Alkylphenolen oder höheren Fettalkoholen mit Ethylenoxid (Fettalkoholethoxylate), die Kondensationsprodukte von Polypropylenglykol mit Ethylenoxid oder Propylenoxid, die Kondensationsprodukte von Ethylenoxid mit dem Reaktionsprodukt aus Ethylendiamin und Propylenoxid, sowie langkettige tertiäre Aminoxide

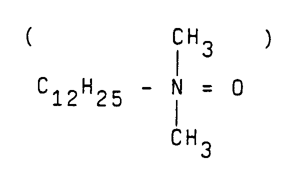

Schließlich umfassen Tenside mit zwitterionischem (ampholytischem) Charakter folgende Verbindungen:

Derivate von aliphatischen, sekundären und tertiären Aminen oder quaternären Ammoniumverbindungen mit 8 bis 18 C-Atomen und einer hydrophilen Gruppe im aliphatischen Rest, wie z.B. Natrium-3-dodecylaminopropionat, Natrium-3-dodecylaminopropansulfonat, 3-(N,N-Dimethyl-N-hexadecyl-ammonium)propan-1-sulfonat oder Fettsäureaminoalkyl-N,N-dimethylacetobetain, wobei die Fettsäure 8 bis 18 C-Atome und der Alkylrest 1-3 C-Atome enthält.

Als Waschhilfsstoffe gemäß der Erfindung eignen sich anorganische Salze, organische Säuren und deren Salze sowie Komplexbildner.

Brauchbare anorganische Salze sind beispielsweise die Bicarbonate oder Carbonate der Alkalien, z.B. Soda. Zu den organischen Waschhilfsstoffen gehören 1 bis 7 C-Atome enthaltende Sulfonsäuren, Carbonsäuren und Sulfocarbonsäuren sowie deren Salze. Hierbei sind vor allem Benzol-, Toluol- oder Xylolsulfonsäure, Sulfoessigsäure, Sulfobenzoesäure, Sulfodicarbonsäuren, Essigsäure, Milchsäure, Zitronensäure und Weinsäure sowie deren Salze hervorzuheben. Zu den Komplexbildnern gehören beispielsweise Nitrilotrieessigsäure, Ethylendiamintetraessigsäure, N-Hydroxyethylethylendiamintriessigsäure oder Polyalkylenpolyamin-N-polycarbonsäuren.

Waschhilfsstoffe gemäß der Erfindung umfassen ferner Produkte wie die Alkali- oder Ammoniumsalze der Schwefelsäure (z.B. Na₂SO₄), Borsäure, Alkylen-, Hydroxyalkylen- oder Aminoalkylenphosphonsäure sowie Bleichmittel, Aktivatoren und Stabilisatoren für Peroxidverbindungen (Bleichmittel).

Im einzelnen gehören zu den Bleichmitteln Natriumperboratmono- oder - tetrahydrat, Na-Percarbonat, die Alkalisalze der Peroxomono- oder Peroxodischwefelsäure, die Alkalisalze der Peroxodiphosphorsäure (H₄P₂O₈) und Alkalisalze von Peroxocarbonsäuren, wie Diperoxododekandisäure. Als Stabilisatoren für diese Bleichmittel fungieren z.B. wasserlösliches, gefälltes Magnesiumsilikat, organische Komplexbildner wie die Alkalisalze der Iminodiessigsäure, Nitrilotriessigsäure, Ethylendiamintetraessigsäure, Methylendiphosphonsäure, 1-Hydroxyethan-1,1-diphosphonsäure und Nitrilotrismethylenphosphonsäure. Als Aktivator für Bleichmittel wird Tetraacetylethylendiamin (TAED) eingesetzt.

Waschhilfsstoffe, die das Schmutztragevermögen von Waschflotten erhöhen, wie Carboxymethylcellulose, Carboxymethylstärke und Methylcellulose, Schaumregulatoren, wie Mono- und Dialkylphosphorsäureester mit 16 bis 20 C-Atomen im Alkylrest sowie optische Aufheller, Desinfizienzien und Enzyme, wie Proteasen, Amylasen, Lipasen, ferner Duftstoffe, können ebenfalls zusätzliche Bestandteile von Waschmitteln sein.

Die sehr guten waschtechnischen Eigenschaften der Polyepoxybernsteinsäure und ihrer Derivate sollen anhand nachfolgender Beispiele demonstriert werden.

### Beispiele

| | Versuchsformulierungen (Gew%) | | | | | | | | | |
|---|---|---|---|---|---|---|---|---|---|---|
| Inhaltsstoffe | 1 | 2 | 3 | 4 | 5 | 6 | 7 | 8 | 9 | 10 |
| Schichtsilikat | 39 | 39 | 39 | 39 | 39 | 15 | 15 | 15 | 15 | 15 |
| δ-Na₂Si₂O₅ | | | | | | | | | | |
| Zeolith A | - | - | - | - | - | 28 | 28 | 28 | 28 | 28 |
| lineare Alkylbenzolsulfonate (LAS) | 11 | 11 | 11 | 11 | 11 | 7 | 7 | 7 | 7 | 7 |
| Nichtionische Tenside | 9 | 9 | 9 | 9 | 9 | 6 | 6 | 6 | 6 | 6 |
| PEBS Typ A | - | 2 | - | - | - | - | 4 | - | - | - |
| PEBS Typ B | - | - | 2 | - | - | - | - | 4 | - | - |
| PEBS Typ C | - | - | - | 2 | - | - | - | - | 4 | - |
| PEBS Typ D | - | - | - | - | 2 | - | - | - | - | 4 |
| Rest auf 100 % | Bleichmittel, Aktivator, Schaumregulatoren, Enzyme, Aufheller, Parfüm, Natriumsulfat | | | | | | | | | |

| Sekundärwaschwirkung * | | | | | | | | | | |
|---|---|---|---|---|---|---|---|---|---|---|
| Testgewebe | 1 | 2 | 3 | 4 | 5 | 6 | 7 | 8 | 9 | 10 |
| Frottee | 2,5 | 0,8 | 0,7 | 1,0 | 1,1 | 2,2 | 1,1 | 1,0 | 1,2 | 1,3 |
| Baumwolle WFK | 3,2 | 0,7 | 0,8 | 1,1 | 1,2 | 3,5 | 1,6 | 1,9 | 2,0 | 2,2 |

| | | | | | | | | | | |
|---|---|---|---|---|---|---|---|---|---|---|
| * Anorganische Gewebeinkrustierung in Gew% nach 25 Waschzyklen, Waschtemperatur 60 °C, Wasserhärte 18 °d (Härtebereich III), Einlaugenverfahren | | | | | | | | | | |

## Patentansprüche

1. Pulverförmiges Waschmittel, enthaltend 0,5 bis 60 Gew% Gerüststoffe aus der Substanzklasse der Natriumschichtsilikate der Formel NaMSiₓO₂ₓ₊₁ . zH₂O mit M = Na oder H; x = 1,9 bis 4; und z = 0 bis 20, sowie übliche Waschhilfsstoffe und 5 bis 50 Gew% mindestens eines Tensids, dadurch gekennzeichnet, daß die Mischung zusätzlich 0,5 bis 10 Gew% mindestens einer Polyepoxybernsteinsäure der Formel 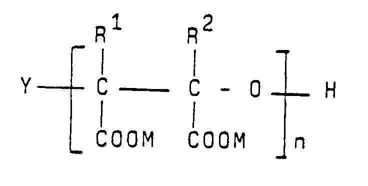 mit R¹, R² = H, C₁₋₄-Alkyl, substituiertes C₁₋₄-Alkyl; M = H, Na, K, NH₄ oder substituiertes Ammonium; Y = -OH, -OR' , -NHR' , -NR'₂, -SR' , -SO₃M (M = H, Na, K) , -PR'₂ mit R' = Alkyl, substituiertes Alkyl, Aryl oder substituiertes Aryl; n = 2 bis 15, enthält.

2. Waschmittel nach Anspruch 1, dadurch gekennzeichnet, daß die Gerüststoffe aus einer Kombination von Schichtsilikat und Zeolith A bestehen.

3. Waschmittel nach Anspruch 1 oder 2, dadurch gekennzeichnet, daß der Gehalt an Gerüststoffen 5 bis 6o Gew%, an Tensiden 10 bis 30 Gew% und an Polyepoxybernsteinsäure 0,5 bis 5 Gew% beträgt.

4. Waschmittel nach Anspruch 1, 2 oder 3, dadurch gekennzeichnet, daß das Gewichtsverhältnis Gerüststoffe zu Polyepoxybernsteinsäure 5 : 1 bis 100 : 1 beträgt.

5. Waschmittel nach einem der Ansprüche 1 bis 4, dadurch gekennzeichnet, daß in der Formel der Polyepoxybernsteinsäure n = 2 bis 10, insbesondere 2 bis 4 bedeutet.

6. Waschmittel nach einem der Ansprüche 1 bis 5, dadurch gekennzeichnet, daß als Gerüststoff aus der Substanzklasse der Natriumschichtsilikate δ -Na₂Si₂O₅ eingesetzt wird.

## Claims

1. A pulverulent washing agent, containing 0.5 to 60 wt.-% bulking agents from the substance class of sodium laminar silicates of formula NaMSiₓO₂ₓ₊₁ . zH₂O where M = Na or H; x = 1.9 to 4; and z = 0 to 20, and common washing adjuvants, and 5 to 50 wt. -% of at least one tenside, characterised in that the mixture also contains 0.5 to 10 wt. -% of at least one polyepoxy succinic acid of formula 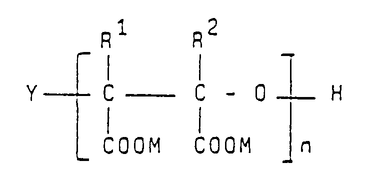 where R¹, R² = H, C₁₋₄ alkyl, substituted C₁₋₄ alkyl; M = H, Na, K, NH₄, or substituted ammonium; Y = -OH, -OR', -NHR', -NR'₂, -SR', -SO₃M (M = H, Na, K), - PR'₂ where R' = alkyl, substituted alkyl, aryl or substituted aryl; n = 2 to 15.

2. A washing agent according to claim 1, characterised in that the bulking agents comprise a combination of laminar silicate and zeolite A.

3. A washing agent according to claim 1 or 2, characterised in that the bulking agents content is 5 - 60 wt. -%, the tenside content is 10 - 30 wt. -% and the polyepoxy succinic acid content is 0.5 to 5 wt. -%.

4. A washing agent according to claim 1, 2 or 3, characterised in that the weight ratio of bulking agents to polyepoxy succinic acid is 5:1 to 100:1.

5. A washing agent according to one of claims 1 to 4, characterised in that in the formula of the polyepoxy succinic acid, n = 2 to 10, especially 2 to 4.

6. A washing agent according to one of claims 1 to 5, characterised in that δ - Na₂Si₂O₅ is used as a bulking agent from the substance class of sodium laminar silicates.

## Revendications

1. Détergent en poudre, contenant 0,5 à 60 % en poids d'adjuvants appartenant à la classe de substances des silicates de sodium lamellaires de la formule NaMSiₓO₂ₓ₊₁ zH₂O, avec M = Na ou H, x = 1,9 à 4 et z = 0 à 20, ainsi que des adjuvants usuels de détergents et 5 à 50 % en poids d'au moins un tensioactif, caractérisé en ce que le mélange renferme en plus 0,5 à 10 % en poids d'au moins un acide polyépoxysuccinique de formule 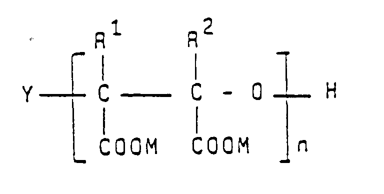 avec R¹, R² = H, un alkyle en C₁₋₄ ou un alkyle en C₁₋₄ substitué; M = H, Na, K, NH₄ ou un ammonium substitué; Y = -OH, -OR', - NHR', -NR'₂, -SR', -SO₃M (M = H, Na, K), -PR'₂ avec R' = un alkyle, un alkyle substitué, un aryle ou un aryle substitué; n = 2 à 15.

2. Détergent selon la revendication 1, caractérisé en ce que les adjuvants sont constitués d'une association de silicate lamellaire et de zéolithe A.

3. Détergent selon la revendication 1 ou 2, caractérisé en ce que la concentration en adjuvants atteint 5 à 60 % en poids, celle en tensioactifs, 10 à 30 % en poids et celle en acide polyépoxysuccinique, 0,5 à 5 % en poids.

4. Détergent selon la revendication 1, 2 ou 3, caractérisé en ce que les adjuvants et l'acide polyépoxysuccinique sont entre eux dans un rapport pondéral de 5:1 à 100:1.

5. Détergent selon une des revendications 1 à 4, caractérisé en ce que dans la formule de l'acide polyépoxysuccinique, n est égal à 2 à 10, en particulier à 2 à 4.

6. Détergent selon une des revendications 1 à 5, caractérisé en ce que l'on met en oeuvre comme adjuvant, un composé appartenant à la classe de substances des silicates de sodium lamellaires de la formule δ-Na₂Si₂O₅.
